(19) Europäisches Patentamt
European Patent Office
Office européen des brevets

(11) Veröffentlichungsnummer: **0 180 732**
**B1**

(12) # EUROPÄISCHE PATENTSCHRIFT

(45) Veröffentlichungstag der Patentschrift: **23.09.87**

(51) Int. Cl.[4]: **C 07 C 147/12**

(21) Anmeldenummer: **85110857.1**

(22) Anmeldetag: **29.08.85**

(54) **Verfahren zur Herstellung von 3-Acylamino-4-alkoxy-phenyl-beta-hydroxysulfonen und -sulfonschwefelsäureestern.**

(30) Priorität: **07.09.84 DE 3432891**

(43) Veröffentlichungstag der Anmeldung: **14.05.86 Patentblatt 86/20**

(45) Bekanntmachung des Hinweises auf die Patenterteilung: **23.09.87 Patentblatt 87/39**

(84) Benannte Vertragsstaaten: **CH DE FR GB IT LI**

(56) Entgegenhaltungen:
**DE-A-3 009 522**
**DE-B-1 443 877**
**DE-C-573 193**
**FR-A-2 461 698**

**CHEMICAL ABSTRACTS, Band 101, Nr. 19, 5. November 1984, Seite 668, Spalte 1, Referat Nr. 170876m, Columbus, Ohio, US**
**CHEMICAL ABSTRACTS, Band 69, Nr. 7, 12. August 1968, Seite 2509, Spalte 2, Referat Nr. 27030w, Columbus, Ohio, US**

(73) Patentinhaber: **HOECHST AKTIENGESELLSCHAFT, Postfach 80 03 20, D-6230 Frankfurt am Main 80 (DE)**

(72) Erfinder: **Hess, Peter, Dr., Am Lorsbacher Kopf 6, D-6238 Hofheim am Taunus (DE)**
Erfinder: **Kohlhaas, Folker, Dr., Akazienring 64, D-6203 Hochheim am Main (DE)**
Erfinder: **Papenfuhs, Theodor, Dr., Heinrich-Bleicher- Strasse 40, D-6000 Frankfurt am Main 50 (DE)**

**EP 0 180 732 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues, gegenüber dem bekannten Verfahren wesentlich verbessertes Verfahren zur Herstellung von 3-(Acyl)amino-4-alkoxyphenyl-β-hydroxyethylsulfon(-schwefelsäureestern) der nachstehenden allgemeinen Formel (1) in hoher Ausbeute und ausgezeichneter Qualität sowie mit erheblich reduzierter Säurelast und organischer Last der Mutterlaugen auf technisch einfache und sicherheitstechnisch unbedenkliche Weise.

Die Verbindungen der nachstehend genannten allgemeinen Formel (1) (mit $R_2$ = H und n = 1) stellen wichtige technische Vorprodukte zur Herstellung faser reaktiver Farbstoffe, wie sie beispielsweise in der DE-OS 3 009 522 (US-PS 4 407 748) beschrieben sind, dar.

Die Herstellung erfolgt bisher nach einem technisch aufwendigen, vom Sicherheitsstandpunkt verbesserungswürdigen Verfahren durch Chlorsulfonierung von o-Nitro-alkoxybenzolen, Reduktion der erhaltenen 2-Nitro-alkoxybenzol-4-sulfochloride mittels Sulfit, Umsetzung der gebildeten 2-Nitro-alkoxybenzol-4-sulfinate mit Ethylenoxid zu den 2-Nitro-alkoxyphenyl-4-β-hydroxyethylsulfonen und deren Reduktion zu den 2-Amino-alkoxyphenyl-4-β-hydroxyethylsulfonen (vgl. JA 67/22767). Abschließend wird die aliphatische Hydroxylgruppe verestert (Überführung der β-Hydroxyethylsulfone in den entsprechenden Schwefelsäurehalbester) (vgl. DE-PS 1 443 877 (US-PS 3 414 579).

Dieses Verfahren ist in mehrfacher Hinsicht technisch unbefriedigend:

1. Die Chlorsulfonierung von 2-Nitro-alkoxybenzolen erfolgt mit Chlorsulfonsäure in Gegenwart von Natriumchlorid bei Temperaturen, die nähe an der Zersetzungstemperatur der Reaktionsmischung liegen. Dadurch wird ein hoher Regelaufwand zur exakten Temperaturführung erforderlich (teuere Apparaturen).

2. Aus Sicherheitsgründen muß die Chlorsulfonierung in hoher Verdünnung, d.h. mit großen Mengen Chlorsulfonsäure durchgeführt werden, wodurch hohe Säure- und Salzlasten der Mutterlaugen resultieren, die eine Entsorgung problematisch machen.

3. Die Ausbeuten bei der Chlorsulfonierung, Reduktion und Ethoxylierung sind nur mäßig, die Qualität der Endprodukte ist teilweise auch dadurch unbefriedigend und es tritt eine hohe organische Last der Abwässer auf, die eine biologische Reinigung sehr aufwendig gestaltet.

Es bestand daher ein dringendes Bedürfnis, die technisch wertvollen Vorprodukte der nachstehenden allgemeinen Formel (1) auf Wegen, die diese Nachteile nicht oder nur in untergeordnetem Maße zeigen, zugänglich zu machen und damit ihre Herstellung umweltfreundlicher und wirtschaftlicher zu gestalten.

Die gestellte Aufgabe wird durch das erfindungsgemäße Verfahren auf äußerst vorteilhafte Weise gelöst.

Es wurde gefunden, daß man die 3-(Acyl)amino-4-alkoxyphenyl-β-hydroxyethylsulfon (-schwefelsäureester) der allgemeinen Formel (1)

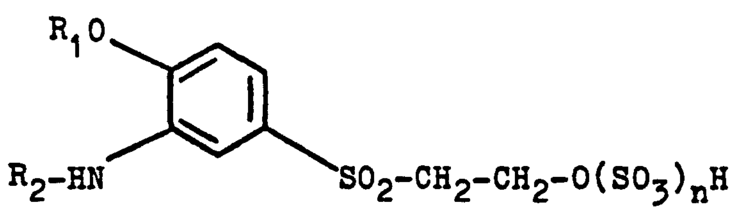

(1)

in welcher $R_1$ eine Alkylgruppe von 1-4 Kohlenstoffatomen, beispielsweise eine Methyl-, Ethyl-, Propyl- oder Butylgruppe bedeutet, $R_2$ ein Wasserstoffatom oder eine Gruppierung der Reihe

Alkyl$_{C_1-C_4}$ CO- , 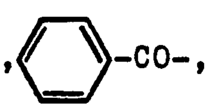, 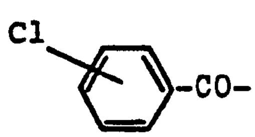, 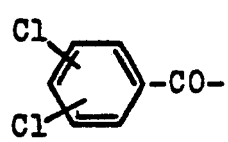,

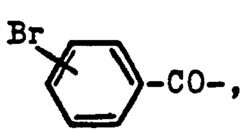, 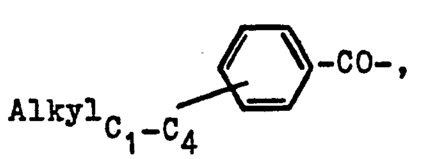, und

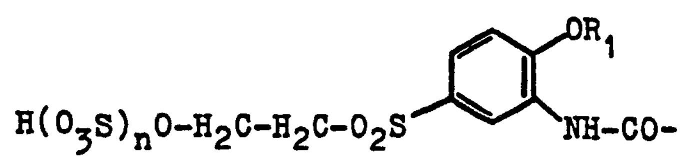

darstellt, mit der Maßgabe, daß n=1 ist, wenn $R_2$ = H ist und n = 0 ist, wenn $R_2$ = Acyl ist, in hoher Ausbeute und in ausgezeichneter Qualität herstellen kann, indem man Alkoxyacylaniline der allgemeinen Formel (2)

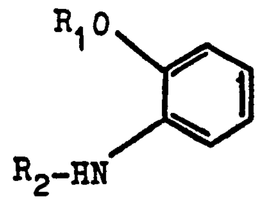

(2)

in welcher $R_1$ und $R_2$ die vorstehend genannten Bedeutungen haben, mit Chlorsulfonsäure in bekannter Weise umsetzt (DE-PS 573 193 (Frdl. 19, 699-701) zu den entsprechenden, in 4-Stellung zur Alkoxygruppe substituierten Sulfochloriden, letztere mit Alkalimetall- oder Ammoniumsulfit in wäßrigem Medium bei einem pH-Wert von 7,0-8,5, vorzugsweise 7,5 - 8,0, bei einer Temperatur von -5 bis +40°C, vorzugsweise 0 - 20°C, reduziert zu den entsprechenden Sulfinaten, diese mit Ethylenoxid in wäßrigem Medium bei einem pH-Wert von 6,0 - 8,5, vorzugsweise 7,0 - 7,5, und einer Temperatur von 40 - 80°C, vorzugsweise 55 - 65 °C, ethoxyliert zu den 3-(Acyl)amino-4-alkoxyphenyl-β-hydroxyethylsulfonen der genannten Formel (1) (mit $R_2$=Acyl und n = 0) und diese ggfs. mit konzentrierter Schwefelsäure bei Temperaturen von 70 - 150°C, vorzugsweise 85 - 135°C, entacylierend verestert zu den Verbindungen der genannten allgemeinen Formel (1) (mit $R_2$=H und n=l).

Dies ist insofern als äußerst überraschend zu erachten, als m-substituierte Benzolsulfochloride bei der Reduktion mit Sulfit nur mäßige Ausbeuten (maximal 60 - 70 %) an den entsprechenden Sulfinaten liefern. Dieser für 3-Nitro-benzol- und 3-Nitro-4-alkoxy-benzolsulfochloride bekannte Tatbestand (siehe weiter oben) wurde in eigenen Untersuchungen auch für 3-Acylaminobenzolsulfochloride bestätigt, indem sich diese mit Sulfit nur zu maximal 55 % zu den 3-Acylaminobenzol-sulfinaten reduzieren lassen.

Es war daher nicht zu erwarten, daß sich 3-Acylamino-4-alkoxybenzolsulfochloride im Gegensatz zu den vorstehend genannten Sulfochloriden mit Sulfit nahezu quantitativ, d.h. in Ausbeuten über 95 %, in die gesuchten 3-Acylamino-4-alkoxybenzolsulfinate überführen lassen.

Die saure Entacylierung der Verbindungen der allgemeinen Formel (1) (mit $R_2$ = Acyl und n = 0) und die Veresterung der hierbei erhaltenen 2-Amino-alkoxybenzol-4-β-hydroxyethylsulfone mit konz. Schwefelsäure zu den Zielverbindungen der Formel (1) (mit $R_2$=H und n=1), sind als Einzelreaktionen zwar bekannt, werden aber beim erfindungsgemäßen Verfahren in einen Reaktionsschritt zusammengefaßt und dadurch besonders wirtschaftlich gestaltet.

Das Verfahren wird im einzelnen in der Weise durchgeführt, daß man das gemäß DE-PS 573 193 frisch hergestellte, wasserfeuchte, oder das anschließend aus einem geeigneten Lösungsmittel trocken isolierte und damit lagerbeständige 2-Acylamino-alkoxybenzol-4-sulfochlorid bei einem pH-Wert von 7,0 - 8,5, vorzugsweise 7,5 - 8,0, in wäßrige Alkalimetallsulfitlösung bei -5 bis +40°C, vorzugsweise 0 bis 20°C, in 0,5 - 2 Stunden einträgt, wobei der pH durch Zudosieren von Alkalimetallhydroxidlösung, vorzugsweise Natron- oder Kalilauge, innerhalb des genannten Bereichs gehalten wird.

Das Sulfit wird in der mindestens stöchiometrischen Menge angewandt. Es ist zweckmäßig, das Sulfit in einem Überschuß von 5 - 30 Molprozent einzusetzen.

Die dabei entstandene Lösung der 2-Acylamino-alkoxybenzol-4-sulfinate wird direkt bei einem pH-Wert von 6,0 - 8,5, vorzugsweise 7,0 - 7,5, und einer Temperatur von 40 - 80°C, vorzugsweise 55 - 65°C, innerhalb 6 - 8 Stunden mit Ethylenoxid (1,5 - 4,5 Mol, vorzugsweise 2,0 - 3,5 Mol pro Mol Sulfinat) versetzt, wobei der pH durch Zudosieren von wäßriger Mineralsäure, vorzugsweise Schwefel- oder Phosphorsäure, im optimalen Bereich gehalten wird. Das Ethylenoxid wird in der mindestens stöchiometrischen Menge eingesetzt.

Das sich dabei kristallin abscheidende 2-Acylaminoalkoxybenzol-4-β-hydroxyethylsulfon wird nach beendeter Reaktion und Abkühlen auf Raumtemperatur durch Filtration in hoher Ausbeute isoliert.

Die entacylierende Veresterung wird vorteilhaft in der Weise durchgeführt, daß man ein Gemisch aus etwa stöchiometrischen Mengen 2-Acylamino-alkoxybenzol-4-β-hydroxyethylsulfon und konzentierter Schwefelsäure (d.h. im Molverhältnis 1:1 bis 1:1,2) in einem technisch üblichen Mischaggregat (wie beispielsweise Trockenpfanne, Kneter, Schaufeltrockner), vorzugsweise unter Vakuum, auf Temperaturen von 70 bis 150°C, vorzugsweise 85 bis 135°C erhitzt und die entstehenden Brüden (Wasser und die abgespaltene Carbonsäure bzw. $CO_2$) aus dem Reaktor abführt. Wenn keine Brüden mehr beobachtet werden, ist die Umsetzung beendet. Im Reaktor befindet sich der in quantitativer Reaktion entstandene Schwefelsäurehalbester des 2-Amino-alkoxyphenyl-hydroxyethylsulfons (Formel (1) mit $R_2$ = H und n = 1) als trockenes, rieselfähiges Pulver, das direkt für Folgereaktionen einsetzbar ist. (Sofern sich in Abhängigkeit des

umzusetzenden Hydroxyethylsulfons ein gewisser Überschuß an konz. Schwefelsäure empfieht, sollte dieser einen 5-molaren Überschuß nicht übersteigen).

Das erfindungsgemäße Verfahren erlaubt es, die Zielverbindungen der genannten Formel (1) auf einem neuen, in allen Stufen mit sehr hohen Ausbeuten ablaufenden Wege in ausgezeichneter Qualität und mit gegenüber dem Stand der Technik erheblich reduzierter Säurelast und organischer Last der Mutterlaugen in technisch einfacher und sicherheitstechnisch unbedenklicher Weise besonders wirtschaftlich herzustellen und stellt damit einen erheblichen technischen Fortschritt dar.

Die nachfolgenden Beispiele sollen das Verfahren näher erläutern, ohne es darauf zu beschränken.

**Beispiel 1**

Zu einer gerührten Mischung aus 303 Teilen 40 %iger wäßriger Natriumhydrogensulfit-Lösung, 143 Teilen 33 %iger wäßriger Natronlauge, 9 Teilen 85 %iger wäßriger Phosphorsäure und 1000 Teilen Wasser von 0°C, die einen pH von 7,5 aufweist, gibt man innerhalb von 30 - 45 Minuten in Anteilen 265 Teile 2-Acetaminoanisol-4-sulfochlorid (z. B. hergestellt gemäß DE-PS 573 193, Beispiel 1). Dabei wird die Temperatur durch Außenkühlung unter 20°C und der pH-Wert durch Zutropfen von insgesamt 246 Teilen 33 %iger wäßriger Natronlauge bei 7,5 - 8,0 gehalten. Anschließend läßt man die Temperatur auf 25 - 30°C ansteigen, wobei im Zeitraum von etwa 1 Stunde alles in Lösung geht.

Dann drückt man unter Rühren 139 Teile flüssiges Ethylenoxid aus einer gekühlten Vorlage unter die Oberfläche der Sulfinatlösung und heizt anschließend auf 60°C. Um ein Entweichen des Ethylenoxids aus dem Reaktionsgefäß zu vermeiden, trägt dieses einen solegekühlten Intensivkühler.

Man rührt 6 - 7 Stunden bei 60 - 70°C nach, wobei der pH-Wert durch Zutropfen von insgesamt 532 Teilen 20 %iger wäßriger Schwefelsäure konstant gehalten wird.

Nach Abkühlen auf Raumtemperatur wird das ausgefallene 2-Acetamino-anisol-4-β-hydroxyethylsulfon abgesaugt und bei 100°C im Umluftschrank getrocknet. Man erhält 297 Teile salzhaltiges Produkt mit einer über HPLC (= High performance liquid chromatography) ermittelten Reinheit von 92,5 %, was einer Ausbeute von 99 % der Theorie, bezogen auf 2-Acetamino-anisol-4-sulfochlorid, entspricht.

**Beispiele 2-6**

Verwendet man aliquote Teile der in der nachstehenden Tabelle aufgeführten 2-Acylamino-alkoxybenzolsulfochloride der Formel (3) (hergestellt beispielsweise nach dem Verfahren der DE-PS 573 193) und arbeitet im übrigen in der in Beispiel 1 angegebenen Weise, eo erhält man die entsprechenden 2-Acylamino-alkoxybenzol-4-β-hydroxyethylsulfone der Formel (4) mit den in der Tabelle I angegebenen Ausbeuten und Reinheiten.

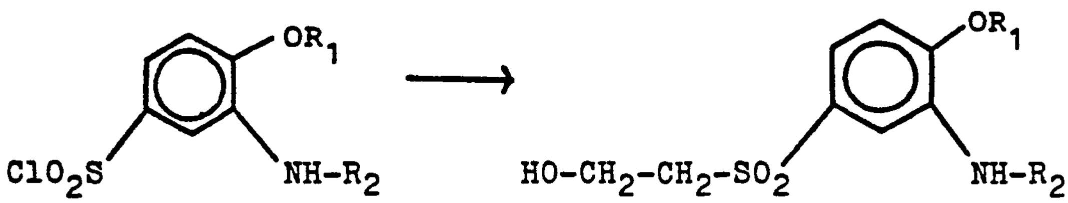

(3) (4)

**Tabelle**

| Beispiel | $R_1$ | $R_2$ | Ausbeute | Reinheit |
|---|---|---|---|---|
| 2 | $C_2H_5$ | $CO-CH_3$ | 97,5 % | 92,8 % |
| 3 | $CH_3$ | A* | 99,1 % | 96,5 % |
| 4 | $CH_3$ | $CO-C_6H_5$ | 98,9 % | 95,8 % |
| 5 | $C_4H_9$ | $CO-CH_3$ | 96,2 % | 90,5 % |
| 6 | $CH_3$ | $CO-C_2H_5$ | 98,0 % | 92,4 % |

* A bedeutet den Rest der Formel

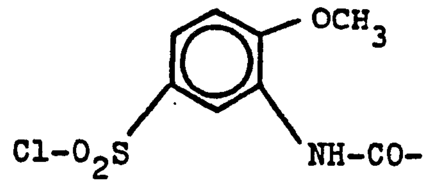

## Beispiel 7

In einem Kneter werden 1200 Teile 2-Acetamino-anisol-4-β-hydroxyethylsulfon 91 %ig (hergestellt nach Beispiel 1) vorgelegt. Dazu läßt man bei laufenden Knetwerkzeugen (Sigmaschaufeln) innerhalb von 15 Minuten 442 Teile 96 %ige Schwefelsäure zulaufen. Man heizt den Trogmantel mit Dampf auf 95-100°C und legt am dichtschließenden Trogdeckel, in dem sich ein Vakuumanschluß befindet, einen Unterdruck von 150-200 Torr an. Unter diesen Bedingungen verläuft die Entacetylierung und parallel dazu die Veresterung innerhalb von 6 - 8 Stunden quantitativ. Die entstandenen Brüden (Wasser und Essigsäure) sind durch den Unterdruck vollständig aus dem Reaktionsgemisch abdestilliert und können in einer zwischen Vakuumerzeuger und Knettrog angebrachten gekühlten Destillatvorlage nahezu quantitativ auskondensiert werden.

Nach der Reaktion wird der Trogmantel bei laufenden Knetwerkzeugen mit Wasser gekühlt und das Reaktionsprodukt aus dem Kneter als nahezu farbloses Pulver isoliert. Man erhält 1290 Teile 2-Amino-anisol-4-β-sulfatoethylsulfon vom Reingehalt (durch Diazotierung) 95,0 %, was einer Ausbeute von 98,6 % der Theorie entspricht.

Verwendet man an Stelle des Kneters ein anderes betriebsübliches Misch- oder Trockenaggregat, beispielsweise eine Trockenpfanne oder einen Schaufeltrockner; und arbeitet im übrigen in der angegebenen Weise, so erhält man das Produkt in vergleichbarer Ausbeute und Qualität.

## Beispiele 8-12

Werden an Stelle von 2-Acetamino-anisol-4-β-hydroxyethylsul-fon aliquote Teile der Verbindungen der Formel (4), wie sie in der nachstehenden Tabelle aufgeführt sind, eingesetzt und wird im übrigen nach der Verfahrensweise des Beispiels 7 gearbeitet, so erhält man die Zielverbindungen der Formel (5) in ebenfalls nahezu quantitativer Ausbeute mit der jeweils in der Tabelle II angegebenen, durch Diazotierung bestimmten Reinheit.

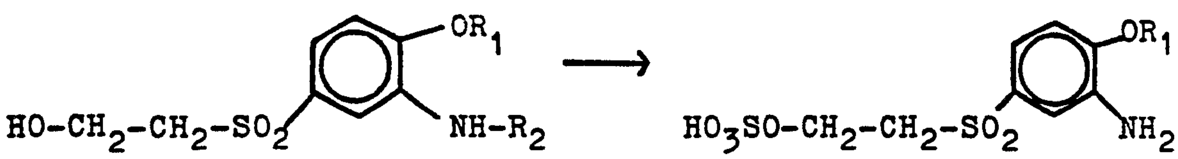

(4) (5)

**Tabelle II**

| Beispiel | $R_1$ | $R_2$ | Reinheit |
|---|---|---|---|
| 8 | $CH_3$ | A* | 97,2 % |
| 9 | $CH_3$ | $-CO-C_2H_5$ | 96,0 % |
| 10 | $CH_3$ | $-CO-C_3H_7$ | 94,2 % |
| 11 | $C_2H_5$ | $-CO-CH_3$ | 95,8 % |
| 12 | $C_4H_9$ | $-CO-CH_3$ | 93,8 % |

* A bedeutet den Rest der Formel

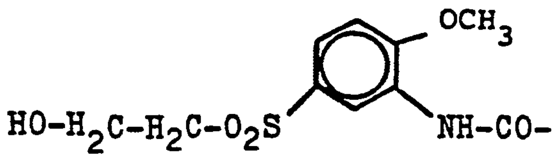

**Patentansprüche**

Verfahren zur Herstellung von 3-(Acyl)amino-4-alkoxyphenyl-β-hydroxyethylsulfon (-schwefelsäureestern) der allgmeinen Formel (1)

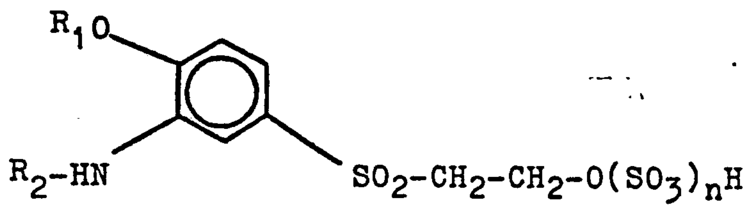

(1)

in welcher $R_1$ eine Alkylgruppe von 1-4 Kohlenstoffatomen, $R_2$ ein Wasserstoffatom oder eine Gruppierung der Reihe

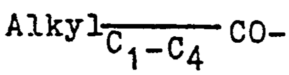 , 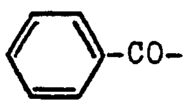, 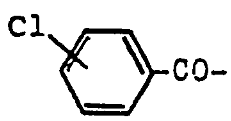, 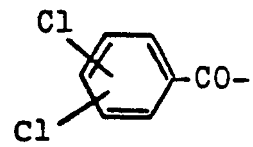,

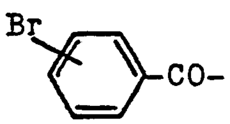, 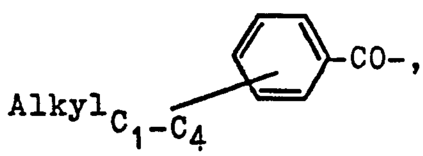, und

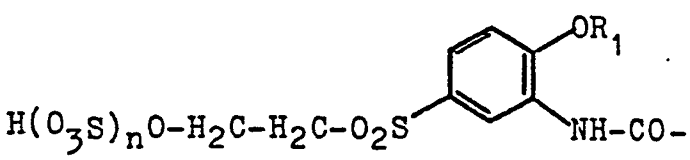

darstellt, mit der Maßgabe, daß n = 1 ist, wenn $R_2$ = H ist, und n = 0 ist, wenn $R_2$ = Acyl ist, dadurch gekennzeichnet daß man 2-Alkoxyacylaniline der allgemeinen Formel (2)

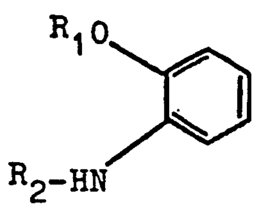

(2)

in welcher $R_1$ und $R_2$ die vorstehend genannten Bedeutungen haben, in bekannter Weise zu den entsprechenden in p-Stellung zur Alkoxygruppe substituierten Sulfochloriden umsetzt, letztere mit Alkalimetall- oder Ammoniumsulfit in wäßrigem Medium bei einem pH-Wert von 7,0 - 8,5 und bei einer

Temperatur von -5 bis +40°C zu den entsprechenden Sulfinaten reduziert, diese mit Ethylenoxid in wäßrigem Medium bei einem pH-Wert von 6,0 - 8,5 und einer Temperatur von 40-80°C ethoxyliert zu den 3-(acyl)amino-4-alkoxyphenyl-β-hydroxyethylsulfonen der genannten Formel (1) (mit $R_2$ = Acyl und n = 0) und diese ggfs. entacylierend verestert mit konz. Schwefelsäure bei einer Temperatur von 70-150°C zu den Verbindungen der genannten allgemeinen Formel (I) (mit $R_2$ = H und n = 1).

**Claim**

A process for the preparation of 3-(acyl)amino-4-alkoxyphenyl-β-hydroxyethyl-sulfone (sulfurie acid esters) of the general formula (I)

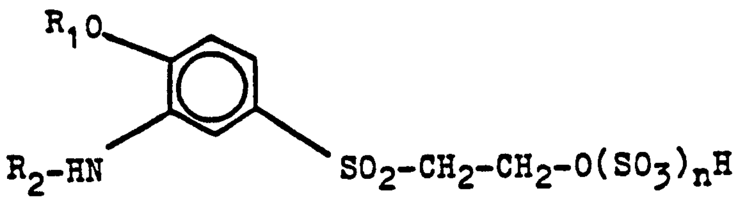

(1)

in which $R_1$ is an alkyl group having 1 - 4 carbon atoms and $R_2$ is a hydrogen atom or a grouping from the series

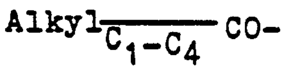 , 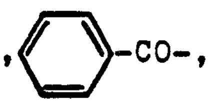 , 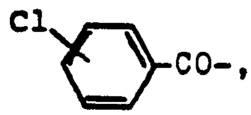 , 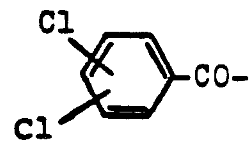

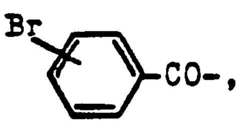 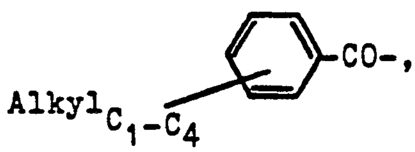 and

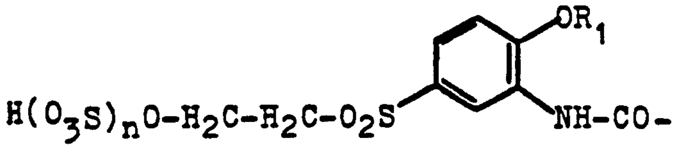

with the proviso that n = 1 if $R_2$ = H and n = 0 if $R_2$ = acyl, which comprises converting 2-alkoxyacylanilines of the formula (2)

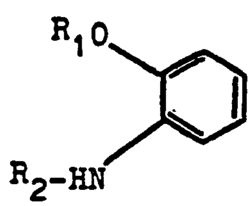

(2)

in which $R_1$ and $R_2$ have the meanings given above, in a known manner to the corresponding sulfochlorides substituted in the p-position relative to the alkoxy group, reducing the sulfochlorides with alkali metal sulfite or ammonium sulfite in an aqueous medium at a pH value of 7.0 - 8.5 and at a temperature of -5 to +40°C to give the corresponding sulfinates, ethoxylating the latter with ethylene oxide in aqueous medium at a pH value of 6.0 - 8.5 and at a temperature of 40 - 80°C to give the 3-acylamino-4-alkoxyphenyl-β-hydroxyethyl-sulfones of the said formula (I) (with $R_2$ = acyl and n = 0) and, if appropriate, esterifying the latter, with deacylation, with concentrated sulfuric acid at a temperature of 70-150°C to give the compounds of the said formula (I) (with $R_2$

= H and n = 1).

## Revendication

Procédé pour préparer des (hydroxy-2-éthylsulfonyl)-1 ou (sulfato-2 éthylsulfonyl)-1 acylamino-3 alcoxy-4 benzènes de formule générale (I)

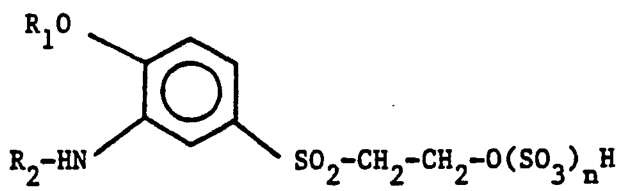

(1)

dans laquelle $R_1$ représente un groupe alkyle ayant 1 à 4 atomes de carbone, $R_2$ représente un atome d'hydrogène ou un groupement de la série:

alkyle $\overline{(C_1-C_4)}$ CO-, 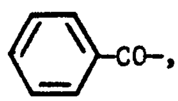 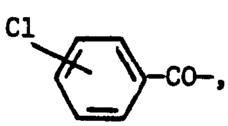 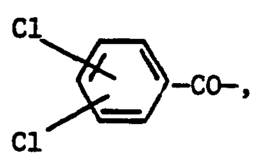

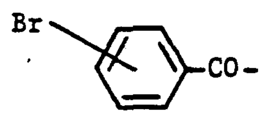 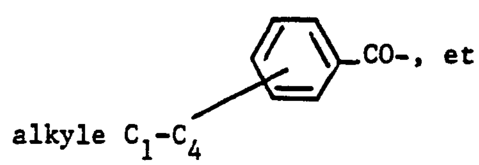

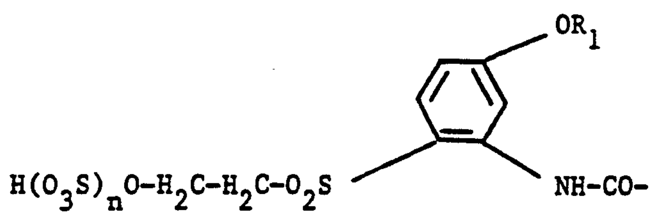

à la condition que n vale 1 quand $R_1$ représente H et que n soit nul quand $R_2$ représente un groupe acyle, procédé caractérisé en ce qu'on fait réagir des alcoxy-2 acylanilines de formule générale (2)

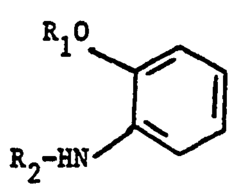

(2)

dans laquelle $R_1$ et $R_2$ ont les sens précités, en opérant de façon connue pour obtenir les sulfochlorures correspondants, substitués en position para du groupe alcoxy, on réduit ces derniers avec un sulfite de métal alcalin ou du sulfite d'ammonium en milieu aqueux à un pH de 7,0 à 8,5 et à une température de -5 à +40°C pour obtenir les sulfinates correspondants, on éthoxyle ces derniers avec de l'oxyde d'éthylène en milieu aqueux à un pH de 6,0 à 8,5 et à une température de 40 à 80°C pour obtenir les hydroxy-β-éthylsulfonyl (acyl)amino-3 alcoxy-4 benzènes de formule (I) précitée (dans laquelle $R_2$ représente un groupe acyle et n est nul) et l'on soumet éventuellement ces derniers composés à une estérification avec désacylation en utilisant de l'acide sulfurique concentré à une température de 70 à 150°C pour obtenir les composés répondant à la formule générale (I) précitée (dans laquelle $R_2$ représente un atome d'hydrogène et n vaut 1).